# EUROPÄISCHE PATENTSCHRIFT

(11) **EP 1 210 054 B1**
(45) Veröffentlichungstag und Bekanntmachung des Hinweises auf die Patenterteilung: **04.08.2004**
(21) Anmeldenummer: 00956455.0
(22) Anmeldetag: 16.08.2000
(51) Int. Cl.: A61K 6/06, C04B 35/486

(54) **ROHLING AUS ZIRKONOXIDKERAMIK MIT OXIDISCHEM ZUSATZ UND DESSEN VERWENDUNG**
BLANK COMPRISED OF A ZIRCONIUM OXIDE CERAMIC WITH AN OXIDIC ADDITIVE AND THE USE THEREOF
EBAUCHE EN CERAMIQUE A L'OXYDE DE ZIRCONIUM A APPORT OXYDIQUE ET SON UTILISATION

(30) Priorität: 16.08.1999 DE 19938143
(43) Veröffentlichungstag der Anmeldung: 05.06.2002
(73) Patentinhaber: 3M ESPE AG, 82229 Seefeld (DE)
(72) Erfinder: HAUPTMANN, Holger, D-82404 Sindelsdorf (DE); SCHNAGL, Robert, D-86899 Landsberg (DE); FRANK, Sybille, D-82229 Seefeld (DE)
(74) Vertreter: Brem, Roland
(86) Internationale Anmeldenummer: PCT/EP2000/007991
(87) Internationale Veröffentlichungsnummer: WO 2001/012132

(56) Entgegenhaltungen:
- EP-A- 0 634 149
- EP-A- 0 908 425
- DE-A- 4 207 179
- DATABASE WPI Section Ch, Week 199927 Derwent Publications Ltd., London, GB; Class L02, AN 1999-323336 XP002153963 & JP 11 116328 A (NGK SPARK PLUG CO LTD), 27. April 1999 (1999-04-27)
- KOSMAC TOMAZ ET AL: "Strength and reliability of surface treated Y-TZP dental ceramics" J BIOMED MATER RES;JOURNAL OF BIOMEDICAL MATERIALS RESEARCH 2000 JOHN WILEY & SONS INC, NEW YORK, NY, USA, Bd. 53, Nr. 4, 2000, Seiten 304-313, XP000972222

## Beschreibung

Die Erfindung betrifft Rohlinge zur Herstellung von Zahnersatz aus Zirkonoxidkeramik mit oxidischem Zusatz. Im besonderen betrifft die Erfindung Rohlinge aus Zirkonoxidkeramik, die mit 0,1 bis 0,50 Gew.-% mindestens eines der Oxide der Elemente Aluminium, Gallium, Germanium, Indium versetzt wurde, zur Herstellung von Zahnersatz, im besonderen von Kronen und Brücken.

Die Verwendung von keramischen Prothesen im Dentalbereich stellt ein wünschenswertes Ziel dar, da keramische Werkstoffe sich durch hohe Festigkeiten auszeichnen und im Gegensatz zu Metallprothesen bereits in der Zahnfarbe vorliegen oder diese nach dem Brennen annehmen. Durch die hohe Gewebeverträglichkeit und die geringere Wärmeleitfähigkeit gegenüber Metallen sind Keramiken besonders für Zahnersatz geeignet.

Reines Zirkonoxid kann nicht für mechanische Anwendungen verwendet werden, da es beim Abkühlprozess nach dem Sintern sein Volumen durch Modifikationsänderungen zu stark verändert. Durch Zugabe von Magnesium-, Cer- oder Yttriumoxid läßt sich dieser Prozess aber eindämmen. Eine ausführliche Diskussion findet sich in "Aluminium- und Zirkonoxidkeramik in der Medizin", Sonderdruck aus Industrie Diamanten Rundschau, IDR 2/1993 sowie in der EP-A-0 634 149.

Die EP-A-0 630 622 beschreibt Rohlinge auf Zirkonoxidbasis, die neben den bei der Verwendung von Zirkonoxid unvermeidbaren Bestandteilen Hafnium- und Yttriumoxid bis zu 0,2 Gew.-% Verunreinigungen aufweisen.

In der EP-A-0 634 149 werden ähnliche Zusammensetzungen beschrieben, wobei der Anteil an Verunreinigungen unter 0,1 Gew.-% betragen soll.

Nachteilig an Verunreinigungen ist die durch diese verursachte Neigung der Keramik zur Glas- bzw. Glasphasenbildung während des Sinterprozesses.

Verunreinigungen wirken sich daher letztlich auf die Festigkeit der aus diesen Keramiken hergestellten Zahnersatzteile negativ aus und sollten unbedingt vermieden werden.

Aufgabe der Erfindung ist es, Rohlinge zur Herstellung von bruchfestem und passgenauem Zahnersatz bereitzustellen.

Überraschenderweise wurde festgestellt, dass Rohlinge, umfassend Keramiken auf Zirkonoxidbasis mit einem Zusatz von 0,1 bis zu 0,50 Gew.-% mindestens eines der Oxide der Elemente Aluminium, Gallium, Germanium, Indium zur Herstellung von komplexem Zahnersatz und filigraner Strukturen geeignet sind. Vorzugsweise liegen die Oxide der oben erwähnten Elemente in einer wie oben definierten Menge mit homogener Verteilung vor und nicht, wie die Verunreinigungen, ungleichmäßig und mit wechselnder Konzentrationsverteilung. Diese homogene Verteilung kann beispielsweise erreicht werden durch Kofällung, wie sie im Ausführungsbeispiel dieser Erfindung beschrieben ist.

Überdies ist eine gleichmäßige Verteilung der während eines Vorsinterprozesses gebildeten Partikel von Vorteil. Die Kornform der Partikel ist hierbei bevorzugt equiaxial mit einem mittleren Korndurchmesser kleiner 1 µm, besonders bevorzugt kleiner 0,7 µm.

Rohlinge gemäß der Erfindung weisen üblicherweise ein Porenvolumen von 50 bis 65 % auf. Die mittlere Porengröße liegt üblicherweise im Bereich von 3 µm bis 0,1 µm, wobei der Bereich von 2 µm bis 0,2 µm bevorzugt ist.

Die erfindungsgemäßen Rohlinge umfassen Keramiken, enthaltend die Komponenten der nachfolgend beschriebenen Zusammensetzung (1):
(A) mindestens 91 Gew.-% Zirkonoxid,
(B) 0 bis 3,5 Gew.-% Hafniumoxid,
(C) 1,5 bis 6,0 Gew.-% Yttriumoxid,
(D) 0,15 bis 0,50 Gew.-% mindestens eines der Oxide der Elemente Aluminium, Gallium, Germanium, Indium,
(E) 0,0005 bis 1,5 Gew.-% färbende Zusätze, ausgewählt aus der Gruppe, bestehend aus den Oxiden Fe₂O₃, Er₂O₃ und/oder MnO₂,
wobei sich die Gew.-% zu 100 ergänzen müssen.

Unter Komponente (E) der Zusammensetzung (1) sind farbende Oxide aus der Gruppe Fe₂O₃, Er₂O₃ oder MnO₂ zu verstehen.

Weitere erfindungsgemäße Rohlinge sind in Ansprüche 2 und 3 definiert.

Die erfindungsgemäßen Rohlinge weisen überdies eine besonders hohe und gleichmäßig verteilte Härte und Festigkeit auf, die sie zur Verwendung bei der Herstellung von hochwertigem Zahnersatz durch Bearbeiten von Rohlingen im nicht-dichtgesinterten Zustand hervorragend geeignet macht.

Da hierbei eine homogene Verteilung der Härte und Festigkeit sowie Dichte innerhalb jeder Raumrichtung des nicht-dichtgesinterten Rohlings benötigt wird, ist es vorteilhaft, dass durch den Einsatz der erfindungsgemäßen Rohlinge auch kleinste Abweichungen in der Dichte- und Härteverteilung der nicht-dichtgesinterten Keramik vermieden werden.

Dies ist insbesondere dann vorteilhaft, wenn filigrane Strukturen oder mehrgliedrige Brücken hergestellt werden sollen, da schon geringste Inhomogenitäten zu Sollbruchstellen führen, die die Haltbarkeit dieser komplexen Strukturen während der Bearbeitung erheblich beeinträchtigen oder zu einem unterschiedlichen Sinterverhalten, welches am Verzug des Werkstückes erkennbar ist, führen.

Der Zusatz von 0,1 bis zu 0,50 Gew.-%, bevorzugt 0,15 bis 0,50 Gew.-%, besonders bevorzugt 0,20 bis 0,50 Gew.-%, ganz besonders bevorzugt 0,25 bis 0,50 Gew.-% mindestens eines der Oxide der Elemente Aluminium, Gallium, Germanium, Indium zu derartigen Keramiken führt zur Erniedrigung der Sintertemperatur und Erhöhung der Stabilität und der hydrolytischen Beständigkeit im dichtgesinterten Zustand. Dieser Sachverhalt findet sich für Aluminiumoxid in der Produktinformation der Firma Tosoh "Zirconia Powder" 09/97 wieder.

Die Verwendung des dort angegebenen Pulvers zur Herstellung passgenauen Zahnersatzes ist nicht angesprochen. Es überrascht vielmehr dessen Eignung für den erfindungsgemäßen Verwendungszweck, da wie vorher ausgeführt, davon ausgegangen werden musste, dass Drittoxide die Bruchfestigkeit negativ beeinflussende Verunreinigungen darstellen und vermieden werden müssen.

Die Herstellung der erfindungsgemäßen Zusammensetzung (1) gelingt durch Auflösen der in käuflichem Zirkonsand enthaltenen Komponenten (A) und (B) der Zusammensetzung (1) mit HCl, mechanischer Abtrennung der schwerlöslichen Verunreinigungen und Vereinigung mit den nach Behandlung mit HCl ebenfalls als Oxichloride bzw. Chloride vorliegenden Additiven (C) und (D) als wässrige, stark saure Lösung.

Färbend wirkende Zusätze gemäß Komponente (E) werden anschließend ebenfalls als Chloride, erhalten durch Auflösung in HCl, zugesetzt.

Es schließt sich eine Kofällung der gelösten Komponenten durch Hydrolyse, Kalzination des Fällungsproduktes, Mahlung des Kalzinates auf die gewünschte Endfeinheit sowie unter Verwendung von temporären Gleit- und Bindemitteln ein Sprühtrockenprozess an.

Die erfindungsgemäßen Rohlinge werden aus dem auf diese Weise erhaltenen Granulat mit bekannten Preßverfahren in die gewünschte Vorform, beispielsweise Zylinder gebracht. Diese Presslinge werden durch eine binderabhängige Wärmebehandlung entbindert und bei einer Temperatur zwischen 850°C und 1000°C, vorzugsweise zwischen 950°C und 995°C mit 0,5 bis 2h Haltezeit vorgesintert.

Die mit gebräuchlichen Verfahren, beispielsweise CAD/CAM oder Kopierfräsen bearbeiteten Rohlinge werden beispielsweise bei 1200°C bis 1650°C, besonders bevorzugt 1350°C bis 1550°C dichtgesintert.

Insbesondere das Vorhandensein von Verunreinigungen in der oben angegebenen Zusammensetzung der erfindungsgemäßen Rohlinge fördert die Entstehung von Glasphasen bzw. Glasanteilen während des Sinterprozesses. Bevorzugt gemäß der vorliegenden Erfindung sind daher Zusammensetzungen, die eine Generierung von Rohlingen erlauben, die während dem Dichtsintem keine Glasphasen bzw. Glasanteile bilden.

Die erfindungsgemäßen Rohlinge weisen ferner eine bevorzugte Abweichung von der Linearität des Schrumpfes pro Raumrichtung auf, die kleiner als 0,05%, besonders bevorzugt kleiner als 0,01 % ist.

Es ist ferner bekannt, dass die Festigkeit von nichtmetallisch-anorganischen Systemen im allgemeinen vom kritischen Spannungsintensitätsfaktor K_{IC} abhängt. Dieser Faktor ist bei amorphen Werkstoffen, beispielsweise Gläsern deutlich niedriger als bei rein kristallinen Systemen (D. Munz/T. Fett: Mechanisches Verhalten keramischer Werkstoffe, Springer-Verlag). Somit sinkt auch die Festigkeit von Keramiken, wenn sich amorphe Phasen an den Korngrenzen bilden. Die erfindungsgemäß bevorzugt einsetzbaren Keramiken weisen daher beispielsweise einen Wert für K_{IC} von 5 bis 10, bevorzugt 8 bis 10, bestimmt gemäß EN 843 auf.

Die Erfindung wird nachfolgend durch Beispiele näher erläutert.

Angaben zu Festigkeiten, insbesondere Bruchfestigkeiten im Rahmen dieser Ausführungen beziehen sich auf den "Punch on three ball Test" gemäß ISO 6872.

Die offenbarten Zirkonoxidkeramiken lassen sich mit besonderem Vorteil im Rahmen des in der DE-199 38 144 beschriebenen Verfahrens zur Herstellung von Zahnersatz mit vorgesinterten Rohlingen anwenden.

Gegenstand der Erfindung sind daher auch Vorrichtungen, die mindestens einen erfindungsgemäßen Rohling enthalten. Unter Vorrichtungen sind beispielsweise offene oder geschlossene Rohlingshalterungen zu verstehen.

Zur Herstellung der erfindungsgemäßen Rohlinge wird von unter Anwendung von Druck erhaltenen Vorkörpern ausgegangen. Bei Herstellung dieser Vorkörper wird beispielsweise von reinen Chloriden, Oxichloriden oder Nitraten ausgegangen, in den Beispielen werden Chloride eingesetzt.

Gegenstand der Erfindung ist auch die Herstellung eines Rohlings gemäß Anspruch 5.

### Herstellungsbeispiele 1 und 2

### Zirkonoxidkeramik mit Aluminiumoxidanteil

Um ca. 200 g fertigdotiertes Pressgranulat zu erhalten, werden die Komponenten gemäß folgender Tabelle in destilliertem Wasser gelöst:

| **Nr.** | **M(ZrCl**_{**4**}**)** **[g]** | **M(YCI** _{**3**}**·6 H**_{**2**}**O)** **[g]** | **M(AlCl**_{**3**}**)** **[g]** | **M(FeCl**_{**3**}**)** **[g]** | **M(ErCl**_{**3**}**)** **[g]** |
|---|---|---|---|---|---|
| **1 [Gefärbt]** | 355,6 | 33,4 | 0,65 | 0,77 | 0,29 |
| (%-Anteil als Oxid) | (94,0) | (5,17) | (0,25) | (0,2) | (0,38) |
| **2 [Ungefärbt]** | 357,66 | 33,36 | 0,65 | 0 | 0 |
| (%-Anteil als Oxid) | (94,55) | (5,20) | (0,25) | | |
| | | | | | |
| **Komponente** | (A) | (C) | (D) | (E) | (E) |

Es schließt sich eine Kofällung der gelösten Komponenten durch Hydrolyse an, wobei die vorgenannte Lösung mit 32 l 6-molarer wässriger NH₄OH-Lösung versetzt wird. Dabei ist ein mindestens 30-facher Überschuß der OH⁻-Konzentration gegenüber dem stöchiometrischen Bedarf empfohlen. Das Fällungsprodukt muss anschließend Cl⁻-frei gewaschen werden. Die Kalzination des Fällungsproduktes erfolgt bei 700°C über 0,75 Stunden, gefolgt von einer Mahlung des Kalzinates auf eine Endfeinheit von D₅₀ = 0,6 µm sowie von einem Sprühtrockenprozess unter Verwendung von temporären Gleit- und Bindemitteln (hier: 2,0 Gew.-% PVA, 0,15 Gew.-% Ölsäure bezogen auf Oxidversatz).

Das erhaltene Granulat wird mit einer isostatischen Presse, beispielsweise bei 1500 bis 2500, bevorzugt 1700 bis 2200 bar in Vorkörper der Abmessungen d = 31 mm und I = 150 mm gebracht.

Die Vorkörper werden durch eine Wärmebehandlung (Aufheizrate: 4 K/min bis 650°C, 1 h Haltezeit) entbindert und bei einer Temperatur bei 970°C mit 0,5 h Haltezeit zu den erfindungsgemäßen Rohlingen vorgesintert.

### Verfahrensbeispiele

Zur Herstellung von passgenauen Brücken werden nach den Herstellungsbeispielen 1 und/oder 2 hergestellte Rohlinge mit einem CAD/CAM-System durch Fräsen oder Schleifen bearbeitet und unter den folgenden Parametern, beispielsweise unter Zuhilfenahme einer Vorrichtung aus der DE-199 04 534 dichtgesintert:
Aufheizrate: 10 K/min bis Endtemperatur: 1500°C
Haltezeit bei Endtemperatur: 2 h

Das Ergebnis ist in beiden Fällen ein extrem passgenauer Zahnersatz mit hoher Bruchfestigkeit (σ > 1000 MPa).

## Patentansprüche

1. Rohling, umfassend Keramik auf Zirkonoxidbasis, enthaltend:
(A) mindestens 91 Gew.-% Zirkonoxid,
(B) 0 bis 3,5 Gew.-% Hafniumoxid,
(C) 1,5 bis 6,0 Gew.-% Yttriumoxid,
(D) 0,15 bis 0,50 Gew.-% mindestens eines der Oxide der Elemente Aluminium, Gallium, Germanium, Indium,
(E) 0,0005 bis 1,5 Gew.-% färbende Zusätze, ausgewählt aus der Gruppe, bestehend aus den Oxiden Fe₂O₃, Er₂O₃ und/oder MnO₂,
wobei sich die Gew.-% zu 100 ergänzen müssen.

2. Rohling gemäß Anspruch 1, enthaltend:
(A) 91 bis 97,25 Gew.-% Zirkonoxid,
(B) 0 bis 2,5 Gew.-% Hafniumoxid,
(C) 2,5 bis 6 Gew.-% Yttriumoxid,
(D) 0,15 bis 0,50 Gew.-% Aluminiumoxid,
(E) 0,0005 bis 1,5 Gew.-% färbende Zusätze, ausgewählt aus der Gruppe, bestehend aus den Oxiden Fe₂O₃, Er₂O₃ und/oder MnO₂,
wobei sich die Gew.-% zu 100 ergänzen müssen.

3. Rohling gemäß Anspruch 1, enthaltend:
(A) 91 bis 98,20 Gew.-% Zirkonoxid,
(B) 0 bis 2,5 Gew.-% Hafniumoxid,
(C) 1,5 bis 6,0 Gew.-% Yttriumoxid,
(D) 0,25 bis 0,50 Gew.-% Aluminiumoxid,
(E) 0,0005 bis 1,5 Gew.-% färbende Zusätze, ausgewählt aus der Gruppe, bestehend aus den Oxiden Fe₂O₃, Er₂O₃ und/oder MnO₂.

4. Verwendung eines Rohlings nach einem der Ansprüche 1 bis 3 zur Herstellung von Zahnersatz.

5. Herstellung eines Rohlings, umfassend Keramik auf Zirkonoxidbasis, wobei folgende Verfahrensschritte zur Anwendung kommen:
(1) Bereitstellen eines Pulvers oder Granulates, enthaltend:
(A) mindestens 91 Gew.-% Zirkonoxid,
(B) 0 bis 3,5 Gew.-% Hafniumoxid,
(C) 1,5 bis 6,0 Gew.-% Yttriumoxid,
(D) 0,15 bis 0,50 Gew.-% mindestens eines der Oxide der Elemente Aluminium, Gallium, Germanium, Indium,
(E) 0,0005 bis 1,5 Gew.-% färbende Zusätze, ausgewählt aus der Gruppe, bestehend aus den Oxiden Fe₂O₃, Er₂O₃ und/oder MnO₂,
wobei sich die Gew.-% zu 100 ergänzen müssen,
(2) Formung des Granulats oder Pulvers unter Anwendung von Druck zu Vorkörpern,
(3) Wärmebehandlung der Vorkörper.

6. Vorrichtungen, enthaltend mindestens einen Rohling nach mindestens einem der Ansprüche 1 bis 3.

## Claims

1. Blank comprising ceramic on a zirconium oxide basis, containing:
(A) at least 91% by weight of zirconium oxide,
(B) 0 to 3.5% by weight of hafnium oxide,
(C) 1.5 to 6.0% by weight of yttrium oxide,
(D) 0.15 to 0.50% by weight of at least one of the oxides of the elements aluminium, gallium, germanium, indium,
(E) 0.0005 to 1.5% by weight of colouring additives, selected from the group consisting of the oxides Fe₂O₃, Er₂O₃ and/or MnO₂,
it being necessary for the % by weight to add up to 100.

2. Blank according to Claim 1, containing:
(A) 91 to 97.25% by weight of zirconium oxide,
(B) 0 to 2.5% by weight of hafnium oxide,
(C) 2.5 to 6% by weight of yttrium oxide,
(D) 0.15 to 0.50% by weight of aluminium oxide,
(E) 0.0005 to 1.5% by weight of colouring additives, selected from the group consisting of the oxides Fe₂O₃, Er₂O₃ and/or MnO₂,
it being necessary for the % by weight to add up to 100.

3. Blank according to Claim 1, containing:
(A) 91 to 98.20% by weight of zirconium oxide,
(B) 0 to 2.5% by weight of hafnium oxide,
(C) 1.5 to 6.0% by weight of yttrium oxide,
(D) 0.25 to 0.50% by weight of aluminium oxide,
(E) 0.0005 to 1.5% by weight of colouring additives, selected from the group consisting of the oxides Fe₂O₃, Er₂O₃ and/or MnO₂.

4. Use of a blank according to one of Claims 1 to 3 for producing dentures.

5. Production of a blank comprising ceramic on a zirconium oxide basis, the following method steps being used:
(1) preparation of a powder or granulated material containing:
(A) at least 91% by weight of zirconium oxide,
(B) 0 to 3.5% by weight of hafnium oxide,
(C) 1.5 to 6.0% by weight of yttrium oxide,
(D) 0.15 to 0.50% by weight of at least one of the oxides of the elements aluminium, gallium, germanium, indium,
(E) 0.0005 to 1.5% by weight of colouring additives, selected from the group consisting of the oxides Fe₂O₃, Er₂O₃ and/or MnO₂,
it being necessary for the % by weight to add up to 100,
(2) shaping the granulated material or powder to form preliminary bodies under the application of pressure,
(3) heat treatment of the preliminary bodies.

6. Devices containing at least one blank according to at least one of Claims 1 to 3.

## Revendications

1. Ebauche comprenant de la céramique à base d'oxyde de zirconium, contenant :
(A) au moins 91% en poids d'oxyde de zirconium,
(B) 0 à 3,5% en poids d'oxyde d'hafnium,
(C) 1,5 à 6,0% en poids d'oxyde d'yttrium,
(D) 0,15 à 0,50% en poids d'au moins un des oxydes des éléments aluminium, gallium, germanium, indium,
(E) 0,0005 à 1,5% en poids d'additifs colorants, choisis dans le groupe constitué par les oxydes Fe₂O₃, Er₂O₃ et/ou MnO₂,
les % en poids devant se compléter pour former 100.

2. Ebauche selon la revendication 1, contenant
(A) 91 à 97,25% en poids d'oxyde de zirconium,
(B) 0 à 2,5% en poids d'oxyde d'hafnium,
(C) 2,5 à 6% en poids d'oxyde d'yttrium,
(D) 0,15 à 0,50% en poids d'oxyde d'aluminium,
(E) 0,0005 à 1,5% en poids d'additifs colorants, choisis dans le groupe constitué par les oxydes Fe₂O₃, Er₂O₃ et/ou MnO₂,
les % en poids devant se compléter pour former 100.

3. Ebauche selon la revendication 1, contenant :
(A) 91 à 98,20% en poids d'oxyde de zirconium,
(B) 0 à 2,5% en poids d'oxyde d'hafnium,
(C) 1,5 à 6,0% en poids d'oxyde d'yttrium,
(D) 0,25 à 0,50% en poids d'oxyde d'aluminium
(E) 0,0005 à 1,5% en poids d'additifs colorants, choisis dans le groupe constitué par les oxydes Fe₂O₃, Er₂O₃ et/ou MnO₂.

4. Utilisation d'une ébauche selon l'une quelconque des revendications 1 à 3 pour la préparation d'une dent de remplacement.

5. Préparation d'une ébauche, comprenant de la céramique à base d'oxyde de zirconium, les étapes de procédé suivantes étant utilisées:
(1) Préparation d'une poudre ou d'un granulat contenant :
(A) au moins 91% en poids d'oxyde de zirconium,
(B) 0 à 3,5% en poids d'oxyde d'hafnium,
(C) 1,5 à 6,0% en poids d'oxyde d'yttrium,
(D) 0,15 à 0,50% en poids d'au moins un des oxydes des éléments aluminium, gallium, germanium, indium,
(E) 0,0005 à 1,5% en poids d'additifs colorants, choisis dans le groupe constitué par les oxydes Fe₂O₃, Er₂O₃ et/ou MnO₂,
les % en poids devant se compléter pour former 100
(2) façonnage du granulat ou de la poudre avec utilisation de pression en corps préalables,
(3) traitement thermique des corps préalables.

6. Dispositifs contenant au moins une ébauche selon au moins l'une quelconque des revendications 1 à 3.
